# EUROPEAN PATENT APPLICATION

(11) **EP 4 670 740 A1**
(43) Date of publication of application: **31.12.2025**
(21) Application number: 24759719.8
(22) Date of filing: 22.02.2024
(51) Int. Cl.: A61K 47/68, A61K 39/395, A61K 45/06, A61P 35/00

(54) **USE OF DRUG CONJUGATE IN TREATMENT OF TUMOR DISEASE AND METHOD**

(30) Priority: 24.02.2023 CN 202310165198; 15.09.2023 CN 202311195966; 07.02.2024 CN 202410174514
(71) Applicant: Sichuan Kelun-Biotech Biopharmaceutical Co., Ltd., Chengdu, Sichuan 611138 (CN)
(72) Inventor: GE, Junyou, Chengdu, Sichuan 611138 (CN); JIN, Xiaoping, Chengdu, Sichuan 611138 (CN); DIAO, Yina, Chengdu, Sichuan 611138 (CN); YANG, Yalan, Chengdu, Sichuan 611138 (CN); LIU, Gesha, Chengdu, Sichuan 611138 (CN); FU, Yujie, Chengdu, Sichuan 611138 (CN); JING, Dian, Chengdu, Sichuan 611138 (CN); ZHONG, Hongbo, Chengdu, Sichuan 611138 (CN)
(74) Representative: Ipsilon
(86) International application number: PCT/CN2024/077993
(87) International publication number: WO 2024/175042

(57) **Abstract**

Provided are a use of a drug conjugate in the treatment of a tumor diseases and a method. Specifically provided are a use of a bioactive agent conjugate represented by formula (I) in the treatment of HR+/Her2- breast cancer and a method. The formula (I) is as follows: {D-[L₁-(L₂)ₘ₁-(L₃)ₘ₂-(L₄)ₘ₃-E]}_{γ}-A.

## Description

The present application is based on the application with CN application number of 202310165198.8 and application date of February 24, 2023, the application with CN application number of 202311195966.0 and application date of September 15, 2023, and the application with CN application number of 202410174514.2 and application date of February 7, 2024, and claims their priority. The disclosed contents of the three CN applications are incorporated into the present application in their entirety.

### TECHNICAL FIELD

The present application relates to the use of a medicament in treatment of diseases related to abnormal cell activity, including but not limited to tumor diseases, especially unresectable locally advanced or metastatic solid tumors that are refractory according to existing treatment standards, such as tumors that have failed and/or recurred after first-line chemotherapeutics, tumors that have failed and/or recurred after radiotherapy, and/or tumors that have failed and/or recurred after targeted therapeutics.

### BACKGROUND

Cancer is a big burden of global public health. In the United States, cancer is still the second leading cause of death after cardiovascular diseases. In China, the number of cancer cases is also on the rise. In 2019, the number of new cases of malignant tumors in China was about 4.4 million, and the death toll was about 2.624 million. The rising number of cancer patients and deaths will lead to the expansion of the overall size of the tumor treatment market.

Chemotherapy is one of the main means of cancer treatment, but traditional chemotherapeutics are not specific in recognizing tumors, which is easy to injure normal cells and cause serious adverse reactions in patients. In order to improve the survival rate of cancer patients, it is urgent to innovate the treatment methods to match the progress of detection and diagnosis. Although great progress has been made in many indications, the mortality resulting from some of the most refractory solid tumors has not been improved significantly since the 1970s, and more effective treatments with fewer side effects are still needed. Molecular targeted drugs are an important direction of drug design at present.

Monoclonal antibody drugs have the advantages of strong targeting, high specificity and low incidence of serious adverse reactions, but their molecular weight is high and their therapeutic effect is limited when used alone. ADCs (antibody-drug conjugates) are drugs that couple monoclonal antibodies with different numbers of small molecular cytotoxins (effector molecules) through chemical linkers. After entering the body, ADC molecules can bind to antigens on the surface of target cells through the guidance of monoclonal antibodies, and enter the target cells.

The ADC molecules entering the cells can release effector molecules through chemical and/or enzymatic actions to achieve the goal of destroying the target cells. ADC drugs combine the advantages of strong targeting of monoclonal antibodies and high activity of small molecular toxins, which can not only reduce the toxic and side effects of small molecular cytotoxins, but also improve the efficacy of drugs.

At present, a variety of ADC drugs are commercially available worldwide, and their indications cover leukemia, lymphoma and breast cancer, etc. However, there are still some problems in the pharmacokinetics and safety of some ADC drugs, which may lead to serious adverse reactions in patients after use. Also, their response rate for some metastatic, recurrent and/or refractory cancers still needs to be further improved. Therefore, there is still a need to develop use or treatment methods for ADC drugs to treat these metastatic, recurrent, and/or refractory cancers, so as to maximize their efficacy and minimize their toxicity to meet the drug demand of cancer patients.

### SUMMARY

The present invention provides use of a biologically active conjugate represented by formula (I) in preparation of a medicament for treating a tumor disease;

{D-[L₁-(L₂ₘ₁-(L₃)ₘ₂-(L₄)ₘ₃-E]}_{γ}-A Formula (I)

wherein,
L₁ is 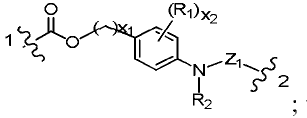 wherein R₁ and R₂ are each independently hydrogen (such as protium or deuterium), halogen, carboxylate, sulfonate, cyano, C₁₋₆ alkyl, halogenated C₁₋₆ alkyl, cyano-substituted C₁₋₆ alkyl (such as-CH₂CN), C₁₋₆ alkoxy, C₂₋₁₀ alkenyl or C₂₋₁₀ alkynyl; Z₁ is an amino acid or a peptide consisting of 2-10 amino acids; x₁ and x₂ are each independently 0, 1, 2, 3, 4, 5 or 6; and the position 1 of L₁ is attached to D, and the position 2 of L₁ is attached to L₂;
L₂ is 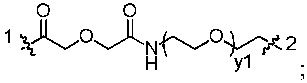 wherein y1 is 0, 1, 2, 3, 4, 5, 6, 7, 8, 9 or 10; and the position 1 of L₂ is attached to L₁, and the position 2 of L₂ is attached to L₃;
L₃ is 5-12-membered heteroaromatic ring;
L₄ is 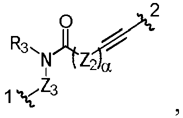 wherein Z₂ is selected from C₁₋₆ alkylene, C₂₋₁₀ alkenylene, C₂₋₁₀ alkynylene, and C₃₋₈ cycloalkylene; R₃ is selected from H and C₁₋₆ alkyl; Z₃ is absent or is C₁₋₆ alkylene; or, R₃ and Z₃ together with the nitrogen atom to which they are attached form 4-8membered heterocyclyl; α is 0, 1, 2, 3, 4, 5 or 6, and the position 2 of L₄ is attached to E, and the position 1 of L₄ is attached to L₃;
E is 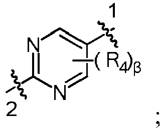 wherein each R₄ is independently hydrogen (such as protium or deuterium), β is 0, 1 or 2, and the position 2 of E is attached to A (such as with sulfhydryl on A), and the position 1 of E is attached to L₄;
m₁, m₂ and m₃ are each independently 0, 1, 2, 3, 4, 5, 6, 7, 8, 9 or 10;
D is a bioactive molecular fragment;
γ refers to the number of moiety {D-[L₁-(L₂)ₘ₁-(L₃)ₘ₂-(L₄)ₘ₃-E}attached to A through a sulfide bond, and is selected from integers from 1 to 10; preferably, γ is selected from integers from 3 to 8 (such as 3, 4, 5, 6, 7 or 8); and
A is a monoclonal antibody against Trop-2 or an antigen-binding fragment thereof.

In some embodiments, the tumor disease is an unresectable locally advanced or metastatic solid tumor that has failed standard treatments, has no standard treatment regimen, or is not suitable for standard treatments at the present stage. In some embodiments, the standard treatment refers to the standard treatment regimens for the tumor disease as recommended by NCCN guidelines and CSCO guidelines for diagnosis and treatment.

In some embodiment, the tumor disease is a tumor that has failed and/or recurred after first line chemotherapeutics. In some embodiments, the first-line chemotherapeutics refer to the first line chemotherapeutics for the tumor disease as recommended by NCCN guidelines and CSCO guidelines for diagnosis and treatment.

In some embodiment, the tumor disease is a tumor that has failed and/or recurred after radiotherapy. In some embodiments, the radiotherapy refers to the radiotherapy regimens for the tumor disease as recommended by NCCN guidelines and CSCO guidelines for diagnosis and treatment.

In some embodiment, that tumor disease is a tumor that has failed and/or recurred after targeted drugs or immunotherapy. In some embodiments, the targeted drug or immunotherapy refers to the targeted drug or immunotherapy for the tumor disease as recommended by NCCN guidelines and CSCO guidelines for diagnosis and treatment.

In some embodiments, the tumor disease includes but is not limited to breast cancer, gastric cancer, lung cancer, ovarian cancer, urinary tract epithelial cancer, esophageal cancer, liver cancer, colorectal cancer, cervical cancer, endometrial cancer, pancreatic cancer, bladder cancer, or brain tumor; preferably breast cancer (such as triple negative breast cancer or Her2 positive breast cancer), ovarian cancer (such as ovarian epithelial cancer), gastric cancer, lung cancer, pancreatic cancer, bladder cancer, or urinary tract epithelial cancer; more preferably, the tumor disease is triple negative breast cancer, Her2 positive breast cancer, ovarian cancer, gastric cancer, lung cancer or pancreatic cancer; further preferably, the tumor disease is triple negative breast cancer, Her2 positive breast cancer, ovarian cancer or gastric cancer.

In some embodiments, the tumor disease is breast cancer.

In some embodiments, the breast cancer includes but is not limited to Luminal A, Luminal B, Her2 positive, triple negative breast cancer or HR+/Her2- breast cancer.

In some embodiments, the breast cancer includes but is not limited to Luminal A, Luminal B, Her2 positive, or triple negative breast cancer.

In some embodiments, the tumor disease is triple negative breast cancer.

In some embodiments, the tumor disease is Her2 positive breast cancer.

In some embodiments, the tumor disease is HR+/Her2- breast cancer.

In some embodiments, the HR+/Her2- breast cancer includes breast cancer without HER2 expression (HER2-zero) or with HER2 low expression (HER2-low).

In some embodiments, the HR+/Her2 breast cancer is HR+/Her2- metastatic breast cancer.

In some embodiments, the HR+/Her2- breast cancer is TROP-2 positive HR+/Her2- breast cancer.

In some embodiments, the HR+/Her2- breast cancer has a TROP-2 expression H-score (H-score) of 0, 0 to 10, 10 to 100, 100 to 200, or more than 200.

In some embodiments, the HR+/HER2- breast cancer disease has a breast cancer susceptibility gene (BRCA) mutation.

In some embodiments, the breast cancer susceptibility gene (BRCA) mutation comprises a BRCA1 and/or BRCA2 mutation.

In some embodiments, the HR+/HER2- breast cancer has a PIK3CA mutation.

In some embodiments, the PIK3CA mutation comprises E542K, E545K, and/or H1047R.

In some embodiments, the HR+/HER2- breast cancer is PD-L1-positive HR+/HER2-breast cancer.

In some embodiments, the PD-L1 expression in the HR+/HER2- breast cancer has a tumor proportion score (TPS) of 1% or more, 5% or more, 10% or more, 20% or more, 30% or more, 40% or more, 50% or more, 60% or more, 70% or more, 80% or more, or 95% or more; or a combined positive score (CPS) of 1 or more, or 10 or more.

In some embodiments, the tumor disease is ovarian cancer.

In some embodiments, the ovarian cancer includes but is not limited to the following types: platinum sensitive type and platinum resistant type.

In some embodiments, the tumor disease is gastric cancer.

In some embodiments, the gastric cancer includes but is not limited to the following types: adenocarcinoma, adenosquamous carcinoma, squamous cell carcinoma, undifferentiated carcinoma and neuroendocrine tumor.

In some embodiments, the tumor disease is pancreatic cancer.

In some embodiments, the pancreatic cancer includes but is not limited to the following types: epithelial tumor, exocrine tumor, borderline tumor, ductal adenocarcinoma, endocrine tumor, mature teratoma, mesenchymal tumor, malignant lymphoma and secondary tumor.

In some embodiments, the tumor disease is bladder cancer.

In some embodiments, the bladder cancer includes but is not limited to the following types: urothelial (transitional cell) carcinoma, squamous cell cancer and adenocarcinoma.

In some embodiments, the tumor disease is urinary tract epithelial cancer.

In some embodiments, the urinary tract epithelial cancer includes but is not limited to the following types: basal type, lumen type and wild type urinary tract epithelial cancers.

In some embodiments, the tumor disease is lung cancer.

In some embodiments, the lung cancer includes but is not limited to the following types: small cell lung cancer and non-small cell lung cancer.

In some embodiments, the conjugate has the following structure:
L₁ is selected from 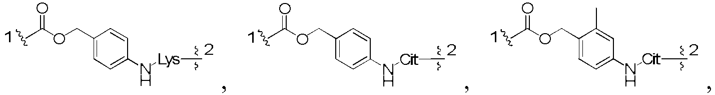 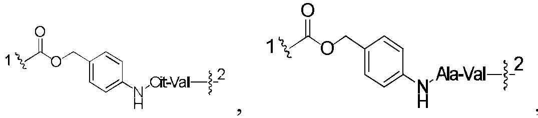 , and 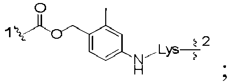 and the position 1 of L₁ is attached to D, and the position 2 of L₁ is attached to L₂;
L₂ is 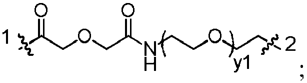 wherein y₁ is 3, 4, 5, 6, 7, 8, 9 or 10; and the position 1 of L₂ is attached to L₁, and the position 2 of L₂ is attached to L₃;
L₃ is selected from 5-6-membered heteroaromatic rings, such as pyrazole or triazole;
L₄ is 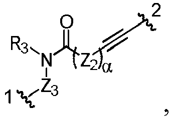 wherein Z₂ is selected from C₁₋₃ alkylene; R₃ is H; Z₃ is selected from C₁₋₃ alkylene; α is 0, and the position 2 of L₄ is attached to E, and the position 1 of L₄ is attached to L₃;
E is 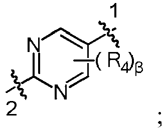 wherein each R₄ is independently hydrogen (such as protium or deuterium), β is 0, 1 or 2, and the position 2 of E is attached to A (such as with sulfhydryl on A), and the position 1 of E is attached to L₄; m₁, m₂ and m₃ are all 1;
the bioactive molecule is selected from 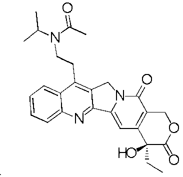 and 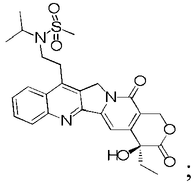 preferably, the bioactive molecule is attached to the position 1 of L₁ through its own hydroxyl; γ is selected from integers from 3 to 8 (for example, 3, 4, 5, 6, 7 or 8);
A is Sacituzumab or an antigen-binding fragment thereof.

In some embodiments, the conjugate has the following structure:
L₁ is selected from 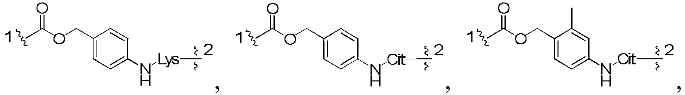 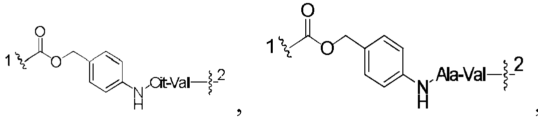 , and 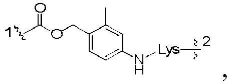 and the position 1 of L₁ is attached to D, and the position 2 of L₁ is attached to L₂;
L₂ is 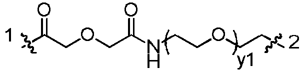 ; wherein y₁ is 3, 4, 5, 6, 7, 8, 9 or 10; and the position 1 of L₂ is attached to L₁, and the position 2 of L₂ is attached to L₃;
L₃ is selected from 5-6-membered heteroaromatic rings, such as pyrazole or triazole;
L₄ is 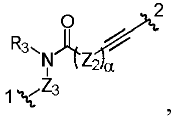 wherein Z₂ is selected from C₁₋₃ alkylene; R₃ is H; Z₃ is selected from C₁₋₃ alkylene; α is 1, and the position 2 of L₄ is attached to E, and the position 1 of L₄ is attached to L₃;
E is 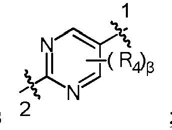 ; wherein each R₄ is independently hydrogen (such as protium or deuterium), β is 0, 1 or 2, and the position 2 of E is attached to A (such as with sulfhydryl on A), and the position 1 of E is attached to L₄; m₁, m₂ and m₃ are all 1;
the bioactive molecule is selected from 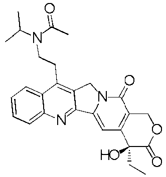 and 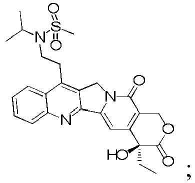 preferably, the bioactive molecule is attached to the position 1 of L₁ through its own hydroxyl; γ is selected from integers from 3 to 8 (for example, 3, 4, 5, 6, 7 or 8); and A is Sacituzumab or an antigen-binding fragment thereof.

In some embodiments, D is selected from 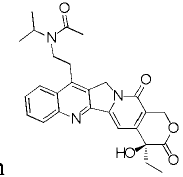 and

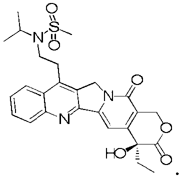

In some technical solutions, the conjugate is conjugate A with a structure represented by the following formula: 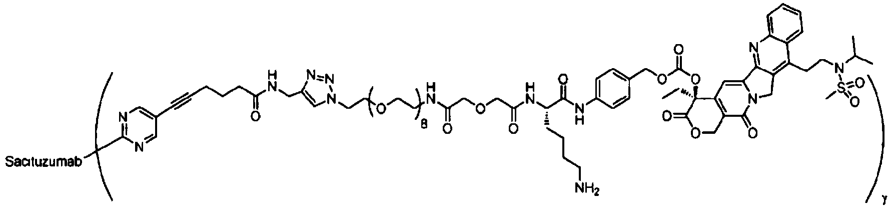 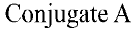 wherein γ is an integer from 1 to 10; preferably, γ is selected from integers from 5 to 8.

In some embodiments, the DAR value of the conjugate is 1-12; preferably 1-10; further preferably, the DAR value is 5-8; for example, the DAR value is 6.1, 6.2, 6.3, 6.4, 6.5, 6.6, 6.7, 6.8, 6.9, 7.0, 7.1, 7.2, 7.3, 7.4, 7.5, 7.6, 7.7, 7.8, 7.9 and 8.0.

The biologically active conjugate shown in the aforementioned Formula (I) of the present invention is used for the treatment of the aforementioned tumor disease.

In another aspect, the present invention provides a method for treating a tumor disease, which comprises a step of administering a therapeutically effective amount of the biologically active conjugate of formula (I) as described above and/or a pharmaceutical composition comprising the biologically active conjugate of formula (I) to an individual in need thereof.

In some embodiments, the tumor disease is an unresectable locally advanced or metastatic solid tumor that has failed standard treatments, has no standard treatment regimen, or is not suitable for standard treatments at the present stage. In some embodiments, the standard treatment refers to the standard treatment regimens for the tumor disease as recommended by NCCN guidelines and CSCO guidelines for diagnosis and treatment.

In some embodiment, that tumor disease is a tumor that has failed and/or recurred after first line chemotherapeutics. In some embodiments, the first-line chemotherapeutics refer to the first-line chemotherapeutics for the tumor disease as recommended by NCCN guidelines and CSCO guidelines for diagnosis and treatment.

In some embodiment, that tumor disease is a tumor that has failed and/or recurred after targeted drugs or immunotherapy. In some embodiments, the targeted drug or immunotherapy refers to the targeted drug or immunotherapy for the tumor disease as recommended by NCCN guidelines and CSCO guidelines for diagnosis and treatment.

In some embodiments, the tumor disease includes but is not limited to breast cancer, gastric cancer, lung cancer, ovarian cancer, urinary tract epithelial cancer, esophageal cancer, liver cancer, colorectal cancer, cervical cancer, endometrial cancer, pancreatic cancer, bladder cancer, or brain tumor; preferably breast cancer (such as triple negative breast cancer or Her2 positive breast cancer), ovarian cancer (such as ovarian epithelial cancer), gastric cancer, lung cancer, pancreatic cancer, bladder cancer, or urinary tract epithelial cancer; more preferably, the tumor disease is triple negative breast cancer, Her2 positive breast cancer, ovarian cancer, gastric cancer, lung cancer or pancreatic cancer; further preferably, the tumor disease is triple negative breast cancer, Her2 positive breast cancer, ovarian cancer or gastric cancer.

In some embodiments, the tumor disease is breast cancer.

In some embodiments, the breast cancer includes but is not limited to the following types: Luminal type A, Luminal type B, Her2 positive type, and triple negative type.

In some embodiments, the tumor disease is triple negative breast cancer.

In some embodiments, the tumor disease is Her2 positive breast cancer.

In some embodiments, the tumor disease is HR-positive and Her2-negative breast cancer (HR+/Her2- breast cancer).

In some embodiments, the HR+/Her2- breast cancer includes breast cancer without HER2 expression (HER2-zero) or with low HER2 expression (HER2-low).

In some embodiments, the HR+/Her2 breast cancer is HR+/Her2- metastatic breast cancer.

In some embodiments, the HR+/Her2- breast cancer is TROP-2 positive HR+/Her2- breast cancer.

In some embodiments, the HR+/Her2- breast cancer has a TROP-2 expression H-score (H-score) of 0, 0 to 10, 10 to 100, 100 to 200, or more than 200.

In some embodiments, the HR+/HER2- breast cancer disease has a breast cancer susceptibility gene (BRCA) mutation.

In some embodiments, the breast cancer susceptibility gene (BRCA) mutation comprises a BRCA1 and/or BRCA2 mutation.

In some embodiments, the HR+/HER2- breast cancer disease has a PIK3CA mutation.

In some embodiments, the PIK3CA mutation comprises E542K, E545K, and/or H1047R.

In some embodiments, the HR+/HER2- breast cancer is PD-L1-positive HR+/HER2-breast cancer.

In some embodiments, the PD-L1 expression in the HR+/HER2- breast cancer has a tumor proportion score (TPS) of 1% or more, 5% or more, 10% or more, 20% or more, 30% or more, 40% or more, 50% or more, 60% or more, 70% or more, 80% or more, or 95% or more; or a combined positive score (CPS) of 1 or more, or 10 or more.

In some embodiments, the tumor disease is ovarian cancer.

In some embodiments, the ovarian cancer includes but is not limited to the following types: platinum sensitive type and platinum resistant type.

In some embodiments, the tumor disease is gastric cancer.

In some embodiments, the gastric cancer includes but is not limited to the following types: adenocarcinoma, adenosquamous carcinoma, squamous cell carcinoma, undifferentiated carcinoma and neuroendocrine tumor.

In some embodiments, the tumor disease is pancreatic cancer.

In some embodiments, the pancreatic cancer includes but is not limited to the following types: epithelial tumor, exocrine tumor, borderline tumor, ductal adenocarcinoma, endocrine tumor, mature teratoma, mesenchymal tumor, malignant lymphoma and secondary tumor.

In some embodiments, the tumor disease is bladder cancer.

In some embodiments, the bladder cancer includes but is not limited to the following types: urothelial (transitional cell) carcinoma, squamous cell cancer and adenocarcinoma.

In some embodiments, the tumor disease is urinary tract epithelial cancer.

In some embodiments, the urinary tract epithelial cancer includes but is not limited to the following types: basal type, lumen type and wild type urinary tract epithelial cancers.

In some embodiments, the tumor disease is lung cancer.

In some embodiments, the lung cancer includes but is not limited to the following types: small cell lung cancer and non-small cell lung cancer. In some embodiments, the pharmaceutical composition comprises the biologically active conjugate and a pharmaceutically acceptable carrier and/or excipient.

In some embodiments, the biologically active conjugate or the pharmaceutical composition is administered once every 7-35 days, preferably once every 7-28 days, such as once every 7 days, 14 days, 21 days, 28 days or 35 days.

In some embodiments, the biologically active conjugate or the pharmaceutical composition is administered once every 14 days.

In some embodiments, the route of administration of the conjugate or pharmaceutical composition includes but is not limited to oral administration, percutaneous injection, rectal administration, mucosal administration, intramuscular injection, intramedullary injection, intravenous injection, or intraperitoneal injection, and preferably intravenous injection.

In some embodiments, the dose of the biologically active conjugate each time based on the body weight of a patient is 1 mg/kg to 30 mg/kg; preferably 1 mg/kg to 20 mg/kg; more preferably 2 mg/kg to 12 mg/kg; further preferably 2-5 mg/kg, 4-7 mg/kg, 6-9 mg/kg, 8-11 mg/kg, 10-13 mg/kg, or 12-15 mg/kg; for example: 2 mg/kg, 2.5 mg/kg, 3 mg/kg, 3.5 mg/kg, 4 mg/kg, 4.5 mg/kg, 5 mg/kg, 5.5 mg/kg, 6 mg/kg, 6.5 mg/kg, 7 mg/kg, 7.5 mg/kg, 8 mg/kg, 8.5mg/kg, 9 mg/kg, 9.5 mg/kg, 10 mg/kg, 11 mg/kg or 12 mg/kg.

In some embodiments, the dose of the biologically active conjugate each time based on the body weight of a patient is 4 mg/kg or 5 mg/kg.

In some embodiments, the dose of the biologically active conjugate for initial administration based on the body weight of a patient is 5 mg/kg, and the tumor disease is HR+/HER2- metastatic breast cancer.

In some embodiments, the individual has primary endocrine resistance.

In some embodiments, the individual has previously received more than two lines of chemotherapy in the metastasis stage.

In some embodiments, the individual has previously received treatments that include taxane therapy and CDK4/6 inhibitor therapy.

In some embodiments, the individual has experienced disease progression after receiving endocrine therapy and at least one chemotherapy for metastatic breast cancer.

In some embodiments, the individual has a tumor tissue with TROP-2-positive expression.

In some embodiments, the individual has a tumor tissue with TROP-2 expression H-score (H-score) of 0, 0 to 10, 10 to 100, 100 to 200, or above 200.

In some embodiments, the individual has a breast cancer susceptibility gene (BRCA) mutation.

In some embodiments, the individual has a breast cancer susceptibility gene (BRCA) mutation and has previously received PARP inhibitor therapy.

In some embodiments, the individual has a breast cancer susceptibility gene (BRCA) mutation comprising BRCA1 and/or BRCA2 mutations.

In some embodiments, the individual has a PIK3CA mutation.

In some embodiments, the individual has a PIK3CA mutation comprising E542K, E545K, and/or H1047R.

In some embodiments, the HR+/HER2- breast cancer of the individual is PD-L1-positive HR+/HER2- breast cancer.

In some embodiments, the tumor proportion score (TPS) of PD-L1 expression in HR+/HER2- breast cancer of the individual is 1% or more, 5% or more, 10% or more, 20% or more, 30% or more, 40% or more, 50% or more, 60% or more, 70% or more, 80% or more, or 95% or more; or the combined positive score (CPS) is 1 or more, or 10 or more.

In some embodiments, the administration regimen of the biologically active conjugate is divided into one or more administration stages (for example, one, two, three or four stages), and the administration cycle and dose at each stage are independently selected from the abovementioned administration cycles or doses.

In some embodiments, the use or method of the present invention results in tumor elimination or volume reduction.

In some embodiments, the use or method of the present invention results in a tumor volume reduction of at least 5%, at least 10%, at least 15%, at least 20%, at least 30% or at least 40%.

### Definitions

Unless otherwise defined below, all technical and scientific terms used herein are intended to have the same meaning as commonly understood by those skilled in the art. References to technologies used herein are intended to refer to technologies commonly understood in the art, including those changes to technologies obvious to those skilled in the art or replacements of equivalent technologies. Although it is believed that the following terms are well understood by those skilled in the art, the following definitions are set forth to better explain the present invention.

Drug-antibody ratio (DAR) refers to the average loading of small molecular toxin drugs by antibodies in conjugates. Although for a specific conjugate molecule, the binding ratio of the small molecule toxin drug moiety to the antibody moiety has an exact value, it should be understood that when used to describe a sample containing many molecules, this value refers to an average value calculated according to the percentages of different specific conjugate molecules, and this average loading is called the average coupling ratio or "DAR" herein.

NCCN guidelines refer to the clinical practice guidelines for various malignant tumors issued by the National Comprehensive Cancer Network.

CSCO guidelines for diagnosis and treatment refer to the guidelines for clinical diagnosis and treatment of various malignant tumors issued by the Chinese Society of Clinical Oncology (CSCO).

Objective Response Rate (ORR) refers to the proportion of patients whose tumors have shrunk to a certain extent and remain at this extent for a certain period of time, including CR and PR cases.

The objective response of tumors is evaluated by Response Evaluation Criteria in Solid Tumors Version 1.1 (RECIST 1.1). Subjects must be accompanied with measurable tumor lesions at baseline. According to the criteria of RECIST 1.1, the evaluation criteria of efficacy are divided into complete response (CR), partial response (PR), stable disease (SD) and progressive disease (PD).

Progressive Disease (PD): with the minimum value in the sums of the diameters of all the measured target lesions as reference, the sum of the diameters is relatively increased by at least 20% (if the baseline measurement value is the minimum, the baseline value will be taken as reference); in addition, the absolute value of the sum of diameters must be increased by at least 5 mm (the appearance of one or more new lesions is also regarded as progressive disease).

Stable Disease (SD): The reduction of the target lesion does not reach PR, and its increase does not reach PD level, the state falls in between, and the minimum sum of diameters could be used as a reference in the study.

Partial Response (PR): The sum of target lesions decreases by at least 30% compared with the baseline.

Complete Response (CR): All target lesions disappear, and the short diameter of all pathological lymph nodes (including target nodules and non-target nodules) must be reduced to <10 mm.

Disease Control Rate (DCR): The percentage of pathologically confirmed cases whose diseases are relived (including complete response and partial response, PR + CR) or stabilized (SD) after treatment among the evaluable cases, under the RECIST criteria of maintaining for at least 4 weeks.

Progression-Free Survival (PFS): The time from randomization to the first occurrence of disease progression or death from any cause.

Dose Limiting Toxicities (DLTs): the toxic and side effects of a drug that become the main reason to limit the continuous increase of drug dose.

Adverse Event (AE): refers to any adverse medical event that occurs after a patient or a clinical research subject receives a drug, but it is not necessarily causally related to the treatment.

Treatment Emergent Adverse Event (TEAE): refers to any AE that occurs or worsens at or after the first dosing.

Duration of Response (DOR): It refers to the time from the first assessment of tumor as CR or PR to the first assessment of PD or death from any cause.

Low expression of HER2 (HER2-low): HER2 immunohistochemistry (IHC) detection result is 1+, or IHC is 2+ and in situ hybridization (ISH) is negative. That is, IHC 1+, or IHC 2+ and ISH-.

### BRIEF DESCRIPTION OF THE DRAWINGS

The drawing described herein is used to provide a further understanding of the present invention and constitute a part of the present application. The illustrative examples of the present invention and their descriptions are used to explain the present invention and do not constitute an undue limitation of the present invention. In the drawings:

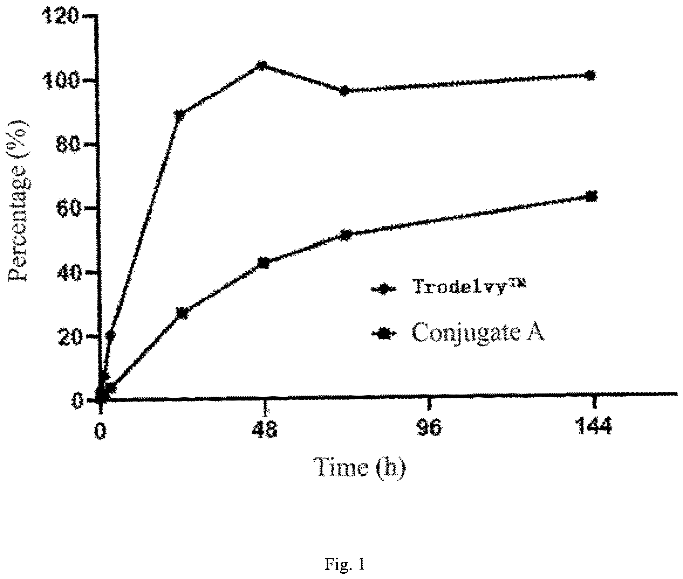

Fig. 1 shows the in vitro plasma stability of the conjugate A and a commercially available drug Trodelvy^{™}.

### DETAILED DESCRIPTION

In the following, the technical solutions in the examples of the present invention will be clearly and completely described with reference to the drawings in the examples of the present invention. Obviously, the described examples are only a part of but not all the examples of the present invention. The following description of at least one exemplary example is merely illustrative in nature and is in no way intended to limit the present invention, its applications or uses. Based on the examples in the present invention, all other examples obtained by those of ordinary skill in the art without creative work fall within the scope of protection of the present invention.

### Example 1. 4-((S)-2-(4-aminobutyl)-35-(4-((6-(2-(methylsulfonyl)pyrimidin-5-yl)hexan-5amido)methyl)-1H-1,2,3-triazol-1-yl)-4,8-dioxo-6,12,15,18,21,24,27,30,33-nonaoxa-3,9diazapentatriacontanamino)benzyl((S)-4-ethyl-11-(2-(N-isopropylmethylsulfonamido)ethyl)3,14-dioxo-3,4,12,14-tetrahydro-1H-pyrano[3',4':6,7]indolizino[1,2-b]quinolin-4-yl)carbonate

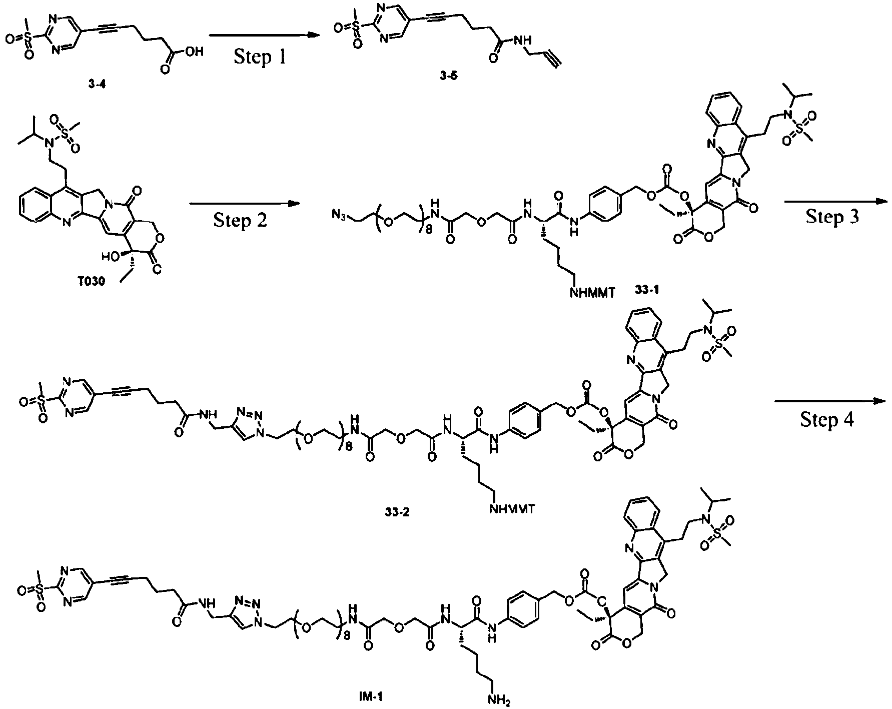

### Step 1: Synthesis of 6-(2-(methylsulfonyl)pyrimidin-5-yl)-N-(propan-2-alkyn-1-yl)hexa-5alkynamide

At 25°C, prop-2-yn-1-amine (189 mg, 3.4 mmol) and compound 3-4 (800 mg, 2.83 mmol) were dissolved in dichloromethane (10 mL), N,N-diisopropylethylamine (738 mg, 5.67 mmol) and O-(7-azabenzotriazol-1-yl)-N,N,N',N'-tetramethylurea hexafluorophosphate (1.63 g, 4.25 mmol) were added in sequence, and the mixture was stirred to react for 2 h. The reaction mixture was concentrated under reduced pressure, and the residue was purified by rapid silica gel column (ethyl acetate/petroleum ether=3/1) to obtain the title compound (700 mg). ESI-MS (m/z): 306.1[M+H]+.

### Step 2: Synthesis of 4-((S)-35-azido-2-(4-((4-methoxyphenyl)diphenylmethyl)amino)butyl)4,8-dioxo-6,12,15,18,21,24,27,30,33-nonaoxa-3,9-diazapentatriacontanamino)benzyl((S)-4ethyl-11-(2-(N-isopropylmethanesulfonamido)ethyl)-3,14-dioxo-3,4,12,14-tetrahydro-2Hpyrano[2,3-b]-1H-pyrano[3',4':6,7]indazino[1,2-b]quinolin-4-yl)carbonate

Under the protection of nitrogen at 25°C, T-030 (250 mg, 0.49 mmol) was dissolved in dichloromethane (10 mL), and cooled to 0°C, then 4-dimethylaminopyridine (478 mg, 3.91 mmol) in dichloromethane (3 mL) was added, and then triphosgene (72 mg, 0.24 mmol) in dichloromethane (10 mL) was slowly added dropwise. Thereafter, the mixture was stirred at 0°C for 20 min and reaction mixture was blown with nitrogen for 20 min. (S)-2-(32-azido-5-oxo3,9,12,15,18,21,24,27,30-nonaoxa-6-azatriacetamidyl)-N-(4-(hydroxymethyl)phenyl)-6(((4methoxyphenyl)diphenylmethyl)amino)acetamide (518 mg, 0.49 mmol) in dichloromethane (7 mL) was added. Thereafter, the mixture was stirred to react for 1 h. The reaction mixture was concentrated under reduced pressure, and the residue was purified by preparative HPLC to obtain the title compound (500 mg). ESI-MS (m/z): 1597.5[M+H]+.

### Step 3: Synthesis of (S)-4-ethyl-11-(2-(N-isopropylsulfonamido)ethyl)-3,14-dioxo-3,4,12,14tetrahydro-1H-pyrano[3',4':6,7]indazino[1,2-b]quinolin-4-yl(4-((S)-2-(4-(((4methoxyphenyl)diphenylmethyl)amino)butyl)-35-(4-((6-(2-(methylsulfonyl)pyrimidin-5yl)hexan-5-amido)methyl)-1H-1,2,3-triazol-1-yl)-4,8-dioxo-6,12,15,18,21,24,27,30,33-nonoxy-3,9-diazapentatriacontanamino)benzyl)carbonate

At room temperature, compound 33-1 (14 mg, 0.05 mmol) was dissolved in dimethyl sulfoxide and water (2.0 mL:0.5 mL), cuprous bromide (11 mg, 0.08 mmol) was added, and the mixture was stirred to react for 1 h. The system was purified by preparative HPLC to obtain the title compound (30 mg). ESI-MS (m/z): 815.9[(M-273)/2+H]+.

### Step 4: Synthesis of 4-((S)-2-(4-aminobutyl)-35-(4-((6-(2-(methylsulfonyl)pyrimidin-5yl)hexan-5-amido)methyl)-1H-1,2,3-triazol-1-yl)-4,8-dioxo-6,12,15,18,21,24,27,30,33-nonaoxa3,9-diazapentatriacontanamino)benzyl((S)-4-ethyl-11-(2-(N-isopropylmethylsulfonamido)ethyl)-3,14-dioxo-3,4,12,14-tetrahydro-1H-pyrano[3',4':6,7]indolizino[1,2-b]quinolin-4-yl)carbonate (compound IM-1)

Compound 33-2 (30 mg, 0.02 mmol) was dissolved in dichloromethane (1.0 mL) and trifluoroacetic acid (0.2 mL) was added to the reaction to react at room temperature for 30 min. After purification by preparative high performance liquid chromatography (method C), the trifluoroacetate of the title compound was obtained (20.0 mg). Its structure is characterized as follows:
¹H NMR (400 MHz, DMSO-d6) δ 10.18 (s, 1H), 9.10 (s, 2H), 8.38 (t, J = 5.56 Hz, 1H), 8.32 (d, J = 8.40 Hz, 1H), 8.22 - 8.20 (m, 2H), 8.09 (t, J = 5.68 Hz, 1H), 7.91-7.87 (m, 2H), 7.82-7.78 (m, 1H), 7.69 (brs, 3H), 7.61 (d, J = 8.56 Hz, 2H), 7.32 (d, J = 8.56 Hz, 2H), 7.06 (s, 1H), 5.56 (d, J = 16.96 Hz, 1H), 5.51 (d, J = 16.96 Hz, 1H), 5.47 (d, J = 19.28 Hz, 1H), 5.42 (d, J = 19.28 Hz, 1H), 5.14 (d, J = 12.20 Hz, 1H), 5.07 (d, J = 12.16 Hz, 1H), 4.48 (t, J = 5.24 Hz, 2H), 4.46 - 4.43 (m, 1H), 4.29 (d, J = 5.60 Hz, 2H), 4.08 - 3.95 (m, 5H), 3.79 (t, J = 5.28 Hz, 2H), 3.51 - 3.43 (m, 32H), 3.40 (s, 3H), 3.39 - 3.35 (m, 2H), 3.30 - 3.26 (m, 2H), 3.00 (s, 3H), 2.82 - 2.74 (m, 2H), 2.56 (t, J = 7.08 Hz, 2H), 2.29 (t, J = 7.36 Hz, 2H), 2.23 - 2.13 (m, 2H), 1.82 (p, J = 7.24 Hz, 2H), 1.78 - 1.63 (m, 2H), 1.61 - 1.49 (m, 2H), 1.42 - 1.27 (m, 2H), 1.15 (d, J = 6.80 Hz, 3H), 1.13 (d, J = 6.76 Hz, 3H), 0.90 (t, J = 7.32 Hz, 3H). ESI-MS (m/z): 816.0[M/2⁺H]⁺. [^{α}]_{D}²⁰ 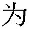 -19.55° (c =1.000g/100mL, CH₃CN).

### Example 2. Preparation of conjugate A

0.3 mL of Sacituzumab antibody (anti-Trop-2, 33.5 mg/mL) was taken, diluted with 0.25 mL of a solution (pH 7.6) containing 20 mM PB, 150 mM NaCl and 20 mM sodium edetate, then 0.45 mL of a solution (pH 7.6) containing 20 mM PB and 150 mM NaCl was added and the mixture was mixed well. pH of the reaction mixture was adjusted to 7.4 with 1 M Na₂HPO₄ solution, 10 mM TCEP (tris(2-carboxyethyl)phosphine) solution was added, the mixture was mixed well, and left to stand at room temperature for 30 min. 10 times in moles of IM-1 trifluoroacetate dissolved in dimethyl sulfoxide was added to the above solution system, the system was mixed well, and left to stand at room temperature for 2 h. Thereafter, 6.1 µl of 100 mM cysteine was added to terminate the reaction. Finally, G-25 gel column was used to replace the buffer solution with PBS buffer solution at pH 6.5, and a product of IM-1 coupled with Sacituzumab antibody was obtained, which was named conjugate A.

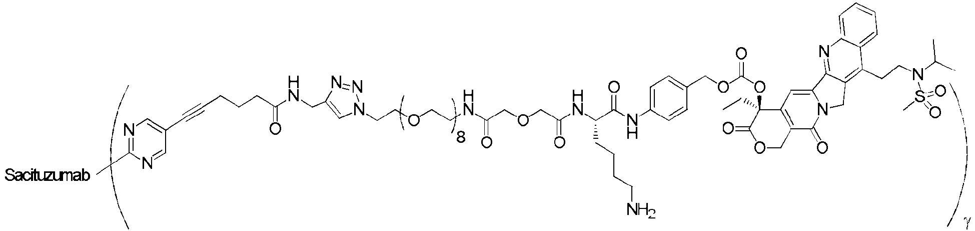

The molecular weight of the conjugate A was analyzed by LCMS, and the measured molecular weight of light chain and heavy chain of the conjugate A was correlated with the theoretical molecular weight of light chain and heavy chain coupled with different numbers of toxins. It was determined that each antibody molecule in the conjugate A was coupled with 1-10 toxins (i.e., γ was 1-10). Then the average coupling ratio (DAR) was calculated to be about 6.9 according to the respective percentages of conjugate molecules coupled with different numbers of toxins.

With reference to Example 2, conjugate A samples with DAR value ranging from 6 to 8 (such as 7.3 or 7.4) were prepared in batches, and the following non-clinical and clinical studies were carried out.

### Experimental Example 1. Detection of the inhibitory effect of conjugate on the proliferation of pancreatic cancer cell line

The effect of the conjugate A on the proliferation of BxPC-3 cells (pancreatic cancer cell line, from ATCC, TROP2 positive cells) was detected by the CellTiter-Glo^{®} chemiluminescence cell viability assay (i.e., CTG). On the first day of the experiment, BxPC-3 cells in exponential growth phase were collected, the concentration of the cell suspension was adjusted with the culture medium, and the suspension was added to a 96- well cell culture plate, the final cell concentration being 2000 cells/well. The cells were cultured overnight in a 5% CO₂ incubator at 37°C. On the second day of the experiment, the conjugate A (final concentration 0.152-1000 nM) was diluted to 10-fold dilute working solution with a gradient of 1:3, and 10 µl of the corresponding 10-fold dilute working solution was added to each well, 3 replicate wells for each drug concentration. After adding the drug, the cells were cultured in a 5% CO₂ incubator at 37°C for 72 h. On the fifth day of the experiment, 50 µl (1/2 culture volume) of CTG solution melted in advance and equilibrated to room temperature was added to each well, and the mixture was mixed well with a microplate shaker for 2 min. After standing at room temperature for 20 min, the fluorescence signal value was measured using an Envision 2104 plate reader.

The results show that the conjugate A significantly inhibited the proliferation of TROP2 positive cell BxPC-3, with the IC₅₀ of 14.3 nM, and the maximum inhibition rate of 95.3%. This indicates that the conjugate A can inhibit the proliferation of TROP2-positive pancreatic cancer cells, suggesting that it has therapeutic effects on pancreatic cancer.

### Experimental Example 2. Detection of the effects of conjugate on cardiovascular and respiratory functions

The cynomolgus monkeys were divided into 10 (half male and half female)/group. The conjugate A was injected intravenously at doses of 25 mg/kg, 50 mg/kg and 75 mg/kg respectively, once every two weeks, for a total of 4 times. The II-lead ECG and respiratory frequency were detected by a large animal noninvasive physiological signal telemetry system, and arterial blood pressure was measured by a noninvasive sphygmomanometer, so as to evaluate the effects of the conjugate A on cardiovascular and respiratory functions of cynomolgus monkeys.

The results show that prior to the first dosing, 2-3 h, 24-25 h, 72-73 h, 7 d after the first dosing, about 2-3 h after the last dosing and prior to the end of the recovery period, for the monkeys in each group receiving the conjugate A, there was no arrhythmia in II-lead ECG, and heart rate, RR interval, P-wave time, P-R interval, QRS wave time, Q-T interval, corrected Q-T interval and other parameters of II-lead electrocardiogram, and systolic blood pressure, diastolic blood pressure, mean arterial pressure and respiratory rate were not significantly abnormal. These results indicate that the conjugate A has no effect on the cardiovascular system and respiratory system of cynomolgus monkeys, and has a good prospect of clinical drug safety.

### Experimental Example 3. Detection of toxic metabolic behavior of conjugate in animals

The cynomolgus monkeys were divided into four groups according to different doses: control group, 25 mg/kg dose group, 50 mg/kg dose group and 75 mg/kg dose group, with 5 males and 5 females in each group. At the dose levels of 25 mg/kg and 50 mg/kg, the cynomolgus monkeys were injected with the conjugate A intravenously once every two weeks for four consecutive times. For the monkeys in each group, blood samples were collected before and immediately after, and 4 h, 24 h, 48 h, 96 h and 168 h after the first and last dosing (0-1 min after the end of dosing), 1 h before and immediately after the second and third dosing (0-1 min after the end of dosing), and 336 h after the last dosing. For the monkeys in the 75 mg/kg dose group, additionally, blood samples were collected at 4 h, 24 h, 48 h, 96 h and 168 h after the third dosing, so as to detect the toxicokinetic behavior of the conjugate and toxin molecules released by it in vivo.

The following table records the peak plasma concentration (Cₘₐₓ) of and exposure (AUC) to the conjugate A and the toxin molecules released by the conjugate A in male and female monkeys after administration in the above manner. The results show that the highest non-serious toxic dose (HNSTD) of the conjugate A was 50 mg/kg, and after the last dosing with this dose, the exposure to the toxin molecules in female and male monkeys was 3.85 h*µg/mL and 5.86 h*µg/mL respectively, and the exposure to the conjugate A in female and male monkeys was 45.8 h*mg/mL and 64.2 h*mg/mL, respectively.

| Dose (mg/kg, QW) | 0 | | 25 | | 50 | | 75 | |
|---|---|---|---|---|---|---|---|---|
| Sex of animal | Male | Female | Male | Female | Male | Female | Male | Female |

| Cₘₐₓ (µg/mL) of conjugate A | | | | | | | | |
|---|---|---|---|---|---|---|---|---|
| Day 1 | NA | NA | 599 | 641 | 1220 | 1170 | 1820 | 2000 |
| Day 43 | NA | NA | 601 | 518 | 1270 | 1370 | 1790 | 1820 |

| Cₘₐₓ(ng/mL) of toxins | | | | | | | | |
|---|---|---|---|---|---|---|---|---|
| Day 1 | NA | NA | 49.0 | 38.3 | 94.0 | 113 | 132 | 132 |
| Day 43 | NA | NA | 64.5 | 65.1 | 146 | 127 | 110 | 137 |

| AUCᵢₐₛₜ (hr*mg/mL) of conjugate A | | | | | | | | |
|---|---|---|---|---|---|---|---|---|
| Day 1 | NA | NA | 25.9 | 26.9 | 63.2 | 46.3 | 87.5 | 85.5 |
| Day 43 | NA | NA | 19.2 | 14.9 | 64.2 | 45.8 | 78.5 | 87.9 |

| AUCₗₐₛₜ (hr*µg/mL) of toxins | | | | | | | | |
|---|---|---|---|---|---|---|---|---|
| Day 1 | NA | NA | 1.79 | 1.43 | 4.49 | 3.97 | 5.42 | 5.12 |
| Day 43 | NA | NA | 2.57 | 1.86 | 5.86 | 3.85 | 4.08 | 4.68 |

According to the experimental results, it can be seen that the exposure or Cₘₐₓ data of ADC and toxins in all of the groups of animals were close after the first and last dosing, indicating that the conjugate A has no obvious toxicity accumulation after continuous intravenous administration, and has good tolerance in animals, so that it has a good prospect for clinical drug safety.

### Experimental Example 4. Metabolic stability of conjugate in vitro

In this experiment, firstly, the conjugate A and Trodelvy^{™} were respectively formulated into a 3.4 mg/mL working solution with physiological saline, and then the conjugate A and Trodelvy^{™} working solutions were added into human blank plasma respectively to obtain 0.05 mg/ml human plasma samples. The plasma samples were incubated at 37°C, and the release of toxin molecules was determined at 1 h, 3 h, 24 h, 48 h, 72 h and 144 h. The conjugate A and the commercially available drug Trodelvy^{™} were compared for the difference in plasma stability in vitro. The results are shown in Fig. 1.

As shown in Fig. 1 of the specification, with the increase of incubation time, the yield of free toxins in human plasma increased, and after incubation for 24 h, the release percentage of toxin molecules of the conjugate A and the commercially available ADC drug Trodelvy^{™} in human plasma was 28.5% and 93.4%, respectively; after incubation for 48 h, the release percentage of toxin molecules of the conjugate A and the commercially available ADC drug Trodelvy^{™} in human plasma was 44.6% and 109.1%, respectively; after incubation for 72 h, the release percentage of toxin molecules of the conjugate A and the commercially available ADC drug Trodelvy^{™} in human plasma was 53.7% and 100.6%, respectively; and after incubation for 144 h, the release percentage of toxin molecules of the conjugate A and the commercially available ADC drug Trodelvy^{™} in human plasma was 65.3% and 104.8%, respectively.

The results showed that the release rate of toxin molecules of conjugate A in human plasma was significantly lower than that of Trodelvy^{™}, which indicates that the conjugate A is relatively stable in human plasma, and releases less toxins in plasma, effectively achieving the goal of releasing toxins after reaching the target tumor sites. Moreover, compared with the commercially available drug Trodelvy^{™}, the conjugate A can reduce the risk of serious adverse reactions caused by rapid and excessive release of toxins in plasma.

### Trial Example 1.

### I. Protocol

Patients with malignant tumor of epithelial origin diagnosed by histology were treated with the conjugate A. All the patients had unresectable locally advanced or metastatic solid tumors that had failed standard treatments, or had no standard treatment regimens or were not suitable for standard treatments at the present stage. Based on the patient's weight, five dose levels of 2, 4, 6, 9 and 12 mg/kg were designed, which were injected intravenously once every two weeks, every 28 days as a cycle, and were given every cycle until the disease progressed or an intolerable toxic reaction occurred. The toxicity of all planned dose groups and some intermediate dose groups was evaluated by BLRM. Based on the model analysis, the sponsor and the researcher jointly decided the decision of dose increase.

### II. Safety results

During the treatment, there was no "Treatment Emergent Adverse Events (hereinafter referred to as TEAEs)" of grade 3 or above in the 2 mg/kg dose group. TEAEs of grade 3 appeared in the 4 mg/kg dose group, including oral mucositis, anemia, etc.; TEAEs of grade 3 or above appeared in the 6 mg/kg dose group, including decreased neutrophil count, reduced white blood cell count, etc.; the abovementioned TEAEs of grade 3 or above can be recovered after symptomatic treatment and the drug can be administered continuously, which shows that the conjugate A has good clinical safety.

### III. Efficacy results

The treatment results for various indications are as follows:

### 1. Triple negative breast cancer (TNBC)

In all the patients with triple negative breast cancer, some patients showed partial response.

A patient who had progressive disease after previous adriamycin/cyclophosphamide/paclitaxel neoadjuvant chemotherapy, left mastectomy and left chest radiotherapy was injected with the conjugate A intravenously at a dose of 4 mg/kg based on the patient's body weight according to the above-mentioned administration cycle. After 18 weeks, partial response was observed, which lasted for 6 weeks. In several times of efficacy evaluation, the total volume of the target lesions could be reduced by 40%. The patient did not have serious adverse events.

### 2. Ovarian cancer

In all the patients with ovarian cancer, some patients showed partial response.

A patient with ovarian cancer who had failed previous hysterectomy, ovarian tumorectomy, carboplatin/paclitaxel, rucaparib, carboplatin/docetaxel/bevacizumab was injected with the conjugate A intravenously at a dose of 4 mg/kg based on the patient's body weight according to the above administration cycle. After 21 weeks, partial response was observed, which lasted for 15 weeks. In several times of efficacy evaluation, the total volume of the target lesions was reduced by 64.8%, and one of the target lesions completely disappeared. The patient did not have serious adverse events.

### 3. HER2 positive breast cancer

A patient with HER2 positive breast cancer who had previously failed modified radical mastectomy, epirubicin/paclitaxel, HER2 monoclonal antibody/docetaxel and capecitabine was injected with the conjugate A intravenously at a dose of 6 mg/kg based on the patient's body weight according to the above administration cycle. After 8 weeks, partial response was observed. In several times of efficacy evaluation, the total volume of the target lesions could be reduced by 49.6%.

### 4. Gastric cancer

A patient with gastric cancer who had previously failed total gastrectomy, paclitaxel/tegafur, anlotinib/tegafur, anlotinib/capecitabine, oxaliplatin/fluorouracil and oxaliplatin/raltitrexed was injected with the conjugate A intravenously at a dose of 4 mg/kg based on the patient's body weight according to the above administration cycle. After 15 weeks, partial response was observed, which had lasted for 10 weeks and was still benefiting. In several times of efficacy evaluation, the total volume of the target lesions could be reduced by 62.8%, and one of the target lesions disappeared.

No serious adverse events occurred in the patient.

### 5. Pancreatic cancer

A patient with pancreatic cancer who had previously failed multi-line therapy of distal resection of pancreatic cancer, radiotherapy, gemcitabine/capecitabine, gemcitabine/irinotecan/fluorouracil, gemcitabine/albumin paclitaxel/oxaliplatin/fluorouracil, and nivolumab/cabiralizumab was injected with the conjugate A intravenously at a dose of 4 mg/kg based on the patient's body weight according to the above-mentioned administration cycle, and stable disease was observed after 7 weeks, which lasted for 30.3 weeks. In several times of efficacy evaluation, the total volume of the target lesions could be reduced by 8.8%, and no serious adverse events occurred in the patient.

To sum up, the clinical trial showed that the conjugate A had a good efficacy at least in the above-mentioned unresectable metastatic TNBC, ovarian cancer, HER2 positive breast cancer, gastric cancer and pancreatic cancer that had failed standard treatments, without inducing serious toxicity that may hinder clinical use. These results prove that the conjugate A has good safety and efficacy in treatment of tumor diseases, and conjugates A with DAR ranging from 6 to 8 (such as DAR of 7.3 or DAR of 7.4) prepared in different batches have basically consistent efficacy and safety.

### Experiment Example 2. Clinical Study of HR+/HER2- Breast Cancer

Patients with HR+/HER2- (including HER2-low and HER2-zero) metastatic breast cancer received Conjugate A at a dose of 5 mg/kg administered every two weeks (Q2W) until disease progression or unacceptable toxicity. Eligibility included progression on endocrine-based therapy and at least one prior chemotherapy for metastatic breast cancer. Tumor assessments were conducted by investigators every 8 weeks according to RECIST v1.1 criteria.

As of April 12, 2023, a total of 41 patients were enrolled, with a median age of 50 years and a median follow-up duration of 8.2 months. Among the 38 patients evaluable for response assessment, 47% had primary endocrine resistance; 79% had previously received ≥2 chemotherapy regimens in the metastasis stage, 100% had prior taxane exposure, and 65.8% had received CDK4/6 inhibitors. The ORR was 36.8% (14/38, including 12 cases confirmed PR and 2 cases unconfirmed PR), the DCR was 89.5%, the DOR was 7.4 months, and the 6-month DOR was 80%. The median PFS was 11.1 months, and the 6-month PFS rate was 61.2%. Treatment-related adverse events (TRAEs) of grade ≥3 were reported in 48.8% (20/41) of the patients. The most common TRAEs (≥5%) of grade ≥3 were decreased neutrophil count (36.6%), decreased white blood cell count (22%), anemia (14.6%), decreased platelet count (13.2%), and increased GGT (7.3%). The treatment-related adverse events (TRAEs) led to dose reductions in 17.1% (7/41) of the patients. No neuropathy or drug-related ILD/pneumonitis was reported. No TRAEs resulted in permanent treatment discontinuation or death.

The enrolled patients were subjected to immunohistochemical research and gene detection research, and it was revealed that some of the enrolled patients had one or more of the following characteristics:
(1) tumors with TROP-2-positive expression;
(2) tumors with PD-L1-positive expression;
(3) having breast cancer susceptibility gene (BRCA) mutations and experienced with prior PARP inhibitor treatment; and/or
(4) having PIK3CA mutation.

### Experiment Example 3. Safety study

In the completed dose-escalation study, Conjugate A was safe and well-tolerated. Twenty-eight (93.9%) patients enrolled in the dose-escalation cohort reported treatment-related adverse events (TRAEs). Seventeen (56.7%) patients experienced grade 3 or above TRAEs, with the most frequent ones (incidence ≥5%) being anemia (26.7%), neutropenia (23.3%), leukopenia (16.7%), stomatitis (16.7%), and thrombocytopenia (13.3%). Patients recovered from all TRAEs of grade 3 or above after corresponding treatment, and no TRAEs resulted in death.

In the dose expansion study, 211 patients received at least one dose of Conjugate A. Twenty-three and 188 patients with different types of advanced solid tumors were treated with Conjugate A at dose levels of 4 mg/kg Q2W and 5 mg/kg Q2W, respectively. TRAEs were reported in 202 (95.7%) patients. Grade 1-2 TRAEs occurred in 101 (47.9%) patients are resolved with supportive care or dose adjustment. Grade 3 or above TRAEs were reported in 110 (52.1%) patients. Treatment-related serious adverse events (TRSAEs) occurred in 51 (24.2%) patients, and no TRAEs led to death. The safety of Conjugate A monotherapy was tolerable and manageable. The table below summarized TRAEs at 4 mg/kg Q2W and 5 mg/kg Q2W dose levels.

### TRAEs at doses of 4 mg/kg Q2W and 5 mg/kg Q2W

| Response type | Conjugate A at 4 mg/kg Q2W (N=23) | | Conjugate A at 5 mg/kg Q2W (N=188) | |
|---|---|---|---|---|
| | All grades (n, %) | Grade ≥3 (n, %) | All grades (n, %) | Grade ≥3 (n, %) |
| Any TRAE | 23 (100) | 11 (47.8) | 179 (95.2) | 99 (52.7) |
| Anemia | 17 (73.9) | 4 (17.4) | 136 (72.3) | 44 (23.4) |
| Lymphopenia | 17 (73.9) | 3 (13.0) | 113 (60.1) | 32 (17.0) |
| Neutropenia | 14 (60.9) | 2 (8.7) | 106 (56.4) | 49 (26.1) |
| Nausea | 10 (43.5) | 0 | 58 (30.9) | 2 (1.1) |
| Vomiting | 10 (43.5) | 0 | 50 (26.6) | 1 (0.5) |
| Stomatitis | 6 (26.1) | 1 (4.3) | 82 (43.6) | 16 (8.5) |
| Alopecia | 3 (13.0) | 0 | 60 (31.9) | 0 (0.0) |
| Rash | 6 (26.1) | 0 | 66 (35.1) | 8 (4.3) |
| Thrombocytopenia | 6 (26.1) | 3 (13.0) | 65 (34.6) | 15 (8.0) |

### Example 4. Pharmacokinetic Study

In the PK analysis of dose-escalation cohort, the exposure of Conjugate A increased proportionally with dose within the tested range of 2 to 6 mg/kg. No accumulation of Conjugate A was observed after multiple administrations. The half-lives of Conjugate A and the free payload were approximately 36 hours and 49 hours, respectively, under the Q2W dosing regimen. The plasma exposure of the free payload (expressed as maximum plasma concentration (Cₘₐₓ) and area under the curve (AUC)) in the first 4-week cycle was about 6% and 5% of that of Conjugate A, respectively, while the pharmacokinetic parameters and plasma exposure of the total antibodies (including conjugated, partially unconjugated, and fully unconjugated antibodies) were similar to those of Conjugate A. These results indicated that the linker of Conjugate A remained stable during systemic circulation, and most payload molecules are guided to tumor tissues by the targeting antibodies.

Various modifications of the present invention other than those described herein will be apparent to those skilled in the art from the foregoing description. Such modifications are also intended to fall within the scope of the appended claims. All the reference documents cited in the present application (including all the patents, patent applications, journal articles, books and any other publications) are incorporated herein by reference in their entirety.

## Claims

1. Use of a biologically active conjugate represented by Formula (1) in the manufacture of a medicament for the treatment of a HR+/Her2- breast cancer;
{D-[L₁-(L₂)ₘ₁-(L₃)ₘ₂-(L₄)ₘ₃-E]}_{γ}-A Formula (I)
wherein,
L₁ is 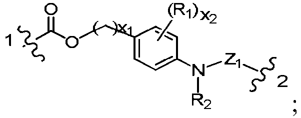
wherein R₁ and R₂ are each independently hydrogen, halogen, carboxylate, sulfonate, cyano, C₁₋₆ alkyl, halogenated C₁₋₆ alkyl, cyano-substituted C₁₋₆ alkyl, C₁₋₆ alkoxy, C₂₋₁₀ alkenyl or C₂₋₁₀ alkynyl; Z₁ is an amino acid or a peptide consisting of 2-10 amino acids; x₁ and x₂ are each independently 0, 1, 2, 3, 4, 5 or 6; and the position 1 of L₁ is attached to D, and the position 2 of L₁ is attached to L₂;
L₂ is 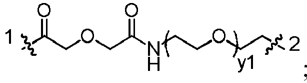 ; wherein y₁ is 0, 1, 2, 3, 4, 5, 6, 7, 8, 9 or 10; and the position 1 of L₂ is attached to L₁, and the position 2 of L₂ is attached to L₃;
L₃ is selected from 5-12-membered heteroaromatic rings;
L₄ is 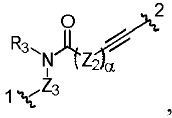 wherein Z₂ is selected from C₁₋₆ alkylene, C₂₋₁₀ alkenylene, C₂₋₁₀ alkynylene, and C₃₋₈ cycloalkylene; R₃ is selected from H and C₁₋₆ alkyl; Z₃ is absent or selected from C₁₋₆ alkylene; or, R₃ and Z₃ together with the nitrogen atom to which they are attached form 4-8-membered heterocyclyl; α is 0, 1, 2, 3, 4, 5 or 6, and the position 2 of L₄ is attached to E, and the position 1 of L₄ is attached to L₃;
E is 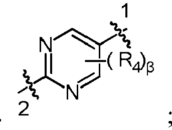 wherein each R₄ is independently hydrogen, β is 0, 1 or 2, and the position 2 of E is attached to A, and the position 1 of E is attached to L₄;
m₁, m₂ and m₃ are each independently 0, 1, 2, 3, 4, 5, 6, 7, 8, 9 or 10; and
D is a bioactive molecular fragment;
γ is selected from integers from 1 to 10; and
A is a monoclonal antibody against Trop-2 or an antigen-binding fragment thereof.

2. The use according to claim 1, wherein the HR+/HER2- breast cancer is an unresectable locally advanced or metastatic breast cancer that has failed standard therapy, or has no standard treatment option, or is currently unsuitable for standard therapy.

3. The use according to claim 1 or 2, wherein the HR+/HER2- breast cancer is of no HER2 expression or low HER2 expression.

4. The use according to any one of claims 1 to 3, wherein the HR+/HER2- breast cancer is HR+/HER2- metastatic breast cancer.

5. The use according to any one of claims 1 to 4, wherein the HR+/HER2- breast cancer is TROP-2-positive HR+/HER2- breast cancer.

6. The use according to any one of claims 1 to 5, wherein the HR+/HER2- breast cancer has a TROP-2 expression H-score of 0, 0 to 10, 10 to 100, 100 to 200, or above 200.

7. The use according to any one of claims 1 to 6, wherein the HR+/HER2- breast cancer disease has a breast cancer susceptibility gene (BRCA) mutation.

8. The use according to claim 7, wherein the BRCA mutation comprises BRCA1 and/or BRCA2 mutations.

9. The use according to any one of claims 1 to 8, wherein the HR+/HER2- breast cancer has a PIK3CA mutation.

10. The use according to claim 9, wherein the PIK3CA mutation comprises E542K, E545K, and/or H1047R.

11. The use according to any one of claims 1 to 10, wherein the HR+/HER2- breast cancer is PD-L1-positive HR+/HER2- breast cancer.

12. The use according to any one of claims 1 to 11, wherein the HR+/HER2- breast cancer has a PD-L1 expression tumor proportion score (TPS) of 1% or higher, 5% or higher, 10% or higher, 20% or higher, 30% or higher, 40% or higher, 50% or higher, 60% or higher, 70% or higher, 80% or higher, or 95% or higher.

13. The use according to any one of claims 1 to 12, wherein the HR+/HER2- breast cancer has a PD-L1 expression combined positive score (CPS) of 1 or higher, or 10 or higher.

14. The use according to any one of claims 1 to 13, wherein the conjugate has the following structure:
L₁ is selected from 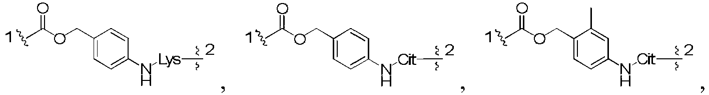 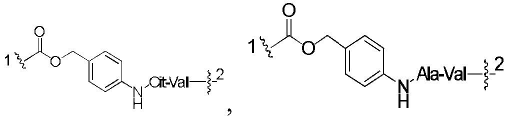 , and 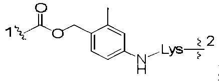 ; and the position 1 of L₁ is 10 attached to D, and the position 2 of L₁ is attached to L₂;
L₂ is 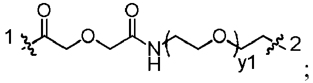
wherein y₁ is 3, 4, 5, 6, 7, 8, 9 or 10; and the position 1 of L₂ is attached to L₁, and the position 2 of L₂ is attached to L₃;
L₃ is selected from 5-6-membered heteroaromatic rings;
L₄ is 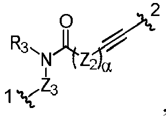 , wherein Z₂ is selected from C₁₋₃ alkylene; R₃ is H; Z₃ is selected from C₁₋₃ alkylene; α is 1, and the position 2 of L₄ is attached to E, and the position 1 of L₄ is attached to L₃;
E is 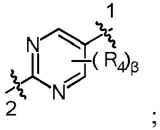 wherein each R₄ is independently hydrogen, β is 0, 1 or 2, and the position 2 of E is attached to A, and the position 1 of E is attached to L₄;
m₁, m₂ and m₃ are all 1;
the bioactive molecule is selected from 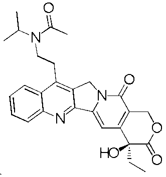
and 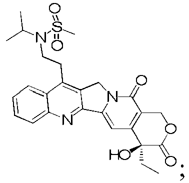
γ is selected from integers from 3 to 8; and
A is Sacituzumab or an antigen-binding fragment thereof.

15. The use according to claim 14, wherein γ is 3, 4, 5, 6, 7 or 8.

16. The use according to any one of claims 1 to 13, wherein the conjugate has the following structure: 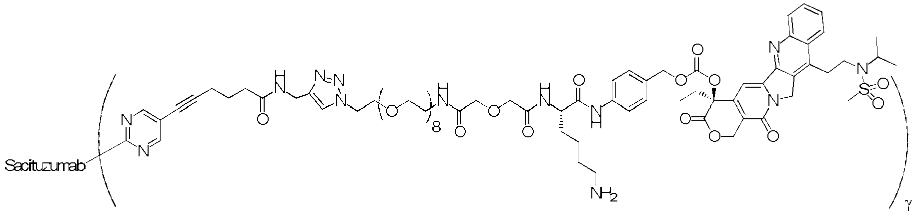 wherein γ is an integer from 1 to 10;

17. The use according to any one of claims 1 to 13, wherein γ is selected from integers from 5 to 8.

18. The use according to any one of claims 1 to 17, the DAR value of the conjugate is 1-12.

19. The use according to any one of claims 1 to 18, the DAR value of the conjugate is 1-10.

20. The use according to any one of claims 1 to 19, the DAR value is 5-8.

21. The use according to any one of claims 1 to 20, the DAR value is 6.1, 6.2, 6.3, 6.4, 6.5, 6.6, 6.7, 6.8, 6.9, 7.0, 7.1, 7.2, 7.3, 7.4, 7.5, 7.6, 7.7, 7.8, 7.9 or 8.0.

22. A method for treating HR+/Her2- breast cancer, wherein the method comprises administering to an individual in need thereof a therapeutically effective amount of the biologically active conjugate according to any one of claims 1, 14 to 21 and/or a pharmaceutical composition comprising the biologically active conjugate according to any one of claims 1, 14 to 21.

23. The method according to claim 22, wherein the HR+/HER2- breast cancer is an unresectable locally advanced or metastatic breast cancer that has failed standard therapy, has no standard treatment option, or is currently unsuitable for standard therapy.

24. The method according to claim 22 or 23, wherein the HR+/HER2- breast cancer comprises breast cancer without HER2 expression or with low HER2 expression.

25. The method according to any one of claims 22 to 24, wherein the HR+/HER2- breast cancer is HR+/HER2- metastatic breast cancer.

26. The method according to any one of claims 22 to 25, wherein the HR+/HER2- breast cancer is TROP-2-positive HR+/HER2- breast cancer.

27. The method according to any one of claims 22 to 26, wherein the HR+/HER2- breast cancer has a TROP-2 expression H-score of 0, 0 to 10, 10 to 100, 100 to 200, or above 200.

28. The method according to any one of claims 22 to 27, wherein the HR+/HER2- breast cancer disease has a breast cancer susceptibility gene (BRCA) mutation.

29. The method according to claim 28, wherein the BRCA mutation comprises BRCA1 and/or BRCA2 mutations.

30. The method according to any one of claims 22 to 29, wherein the HR+/HER2- breast cancer has a PIK3CA mutation.

31. The method according to claim 30, wherein the PIK3CA mutation comprises E542K, E545K, and/or H1047R.

32. The method according to any one of claims 22 to 31, wherein the HR+/HER2- breast cancer is PD-L1-positive HR+/HER2- breast cancer.

33. The method according to any one of claims 22 to 32, wherein the HR+/HER2- breast cancer has a PD-L1 expression tumor proportion score (TPS) of 1% or higher, 5% or higher, 10% or higher, 20% or higher, 30% or higher, 40% or higher, 50% or higher, 60% or higher, 70% or higher, 80% or higher, or 95% or higher.

34. The method according to any one of claims 22 to 33, wherein the HR+/HER2- breast cancer has a PD-L1 expression combined positive score (CPS) of 1 or higher, or 10 or higher.

35. The method according to any one of claims 22 to 34, wherein the pharmaceutical composition comprises the biologically active conjugate and a pharmaceutically acceptable carrier and/or excipient.

36. The method according to any one of claims 22 to 35, wherein the biologically active conjugate or the pharmaceutical composition is administered once every 7 to 35 days.

37. The method according to any one of claims 22 to 36, wherein the biologically active conjugate or the pharmaceutical composition is administered once every 7-28 days.

38. The method according to any one of claims 22 to 37, wherein the biologically active conjugate or the pharmaceutical composition is administered once every 7 days, 14 days, 21 days, 28 days or 35 days.

39. The method according to any one of claims 22 to 38, wherein the biologically active conjugate or the pharmaceutical composition is administered once every 14 days.

40. The method according to any one of claims 22 to 39, wherein the biologically active conjugate or the pharmaceutical composition is administrated by a route of oral, percutaneous injection, rectal administration, transmucosal administration, intramuscular injection, intramedullary injection, intravenous injection, or intraperitoneal injection.

41. The method according to any one of claims 22 to 40, wherein the biologically active conjugate or the pharmaceutical composition is administrated by a route of intravenous injection.

42. The method according to any one of claims 22 to 41, wherein the dose of the biologically active conjugate each time based on the body weight of a patient is 1 mg/kg to 30 mg/kg.

43. The method according to any one of claims 22 to 42, wherein the dose of the biologically active conjugate each time based on the body weight of a patient is 1 mg/kg to 20 mg/kg.

44. The method according to any one of claims 22 to 43, wherein the dose of the biologically active conjugate each time based on the body weight of a patient is 2 mg/kg to 12 mg/kg.

45. The method according to any one of claims 22 to 44, wherein the dose of the biologically active conjugate each time based on the body weight of a patient is 2-5 mg/kg, 4-7 mg/kg, 6-9 mg/kg, 8-11 mg/kg, 10-13 mg/kg, or 12-15 mg/kg.

46. The method according to any one of claims 22 to 42, wherein the dose of the biologically active conjugate each time based on the body weight of a patient is 4mg/kg or 5mg/kg.

47. The method according to any one of claims 22 to 46, wherein the dose of the biologically active conjugate each time based on the body weight of a patient is 5 mg/kg, and the HR+/Her2-breast cancer is HR+/Her2- metastatic breast cancer.

48. The method according to any one of claims 22 to 47, which is divided into one or more administration stages, wherein the administration cycle for each stage is as described in any one of claims 36 to 39, and/or the dose for administration in each stage is as described in any one of claims 42 to 47.

49. The method according to any one of claims 22 to 48, which is divided into one, two, three, or four administration stages.

50. The method according to any one of claims 22 to 49, wherein the individual has primary endocrine resistance.

51. The method according to any one of claims 22 to 50, wherein the individual has previously received more than two lines of chemotherapy in metastasis stage.

52. The method according to claim 51, wherein the individual has received a prior treatment including taxane therapy and CDK4/6 inhibitor therapy.

53. The method according to any one of claims 22 to 52, wherein the individual has experienced disease progression after prior endocrine therapy and at least one chemotherapy for metastatic breast cancer.
